(19) **Europäisches Patentamt / European Patent Office / Office européen des brevets**

(11) **EP 4 767 816 A1**

(12) # EUROPEAN PATENT APPLICATION

(43) Date of publication:
**01.07.2026 Bulletin 2026/27**

(21) Application number: **24307324.4**

(22) Date of filing: **27.12.2024**

(51) International Patent Classification (IPC):
**A01H 1/02** (2006.01)    **C12Q 1/68** (2018.01)
**C12Q 1/6895** (2018.01)

(52) Cooperative Patent Classification (CPC):
**A01H 1/045; A01H 1/023; C12Q 1/6895;**
C12Q 2600/13; C12Q 2600/156

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Limagrain Europe**
**63360 Saint-Beauzire (FR)**

(72) Inventors:
- **VARENNE, Pierrick**
  **30210 LÉDENON (FR)**
- **BOEVEN, Philipp**
  **31234 EDEMISSEN (DE)**
- **POUPARD, Bruno**
  **63720 CHAPPES (FR)**
- **AUDES, Aurélien**
  **63720 CHAPPES (FR)**

(74) Representative: **Flesselles, Bruno F.G.**
**BF IP**
**36 rue Jean de la Fontaine**
**75016 Paris (FR)**

(54) # METHOD FOR ANALYSIS OF MALE POLLINATOR PLANTS

(57)    The invention relates to a method for identifying male pollinator plants, through analysis of specific F1 plants from such male pollinator plants.

EP 4 767 816 A1

**Description**

The invention relates to a method for identifying male pollinator plants, through analysis of specific F1 plants from such male pollinator plants.

The exploitation of heterosis, obtained by breeding male lines with female lines, in wheat and other small-grain cereals like barley holds great potential to increase grain yield and yield stability. Hybrid breeding also offers the opportunity to split major genes with dominant gene action between the two parental pools. This leads to reduced gene stacking efforts compared to line breeding and enables the combination of gametes in the F1 which are linked in repulsion in the parental components. However, many small-grain cereals like wheat or barley possess a cleistogamic nature and it is necessary to be able to select plants for outcrossing ability, which is required to enable a cost-efficient hybrid seed production and to reduce cost of goods.

Good male floral characteristics like extruded anthers releasing enough viable pollen over a long period of time at anthesis are required irrespectively of the used hybrid mechanism that makes the female male sterile: chemical hybridization agents (CHA), cytoplasmic male sterility (CMS), or nuclear genetic male sterility (NGMS). Several indirect traits (anther extrusion, anther filament length, size of anthers, number of pollen grains, viability of pollen grains, plant height, flowering time and time-period of pollen release, etc.) have been evaluated for the selection of male candidates. There is a large variation for trait heritability, for the feasibility of scoring each trait in high-throughput screening.

There is a need to identify male plants that would have good pollinating ability (or good "ability to give seed set", or pollination ability), that would be easy to implement and provide robust and reliable results. Such "ability to give seed set" correspond to the ability to adequately pollinate females and obtain seeds from the female. It can also be named "pollinating ability" in the present application. A male 1 shall be considered as having a higher ability to give seed set than a male 2, when more seeds are obtained after pollination of the same female (or of a set of females) in the same conditions. Currently, for comparing males in their pollinating ability, one would produce each potential hybrid combination in a crossing block, and then measure how many F1 seed can be harvested for each crossing block. However, such a method is expensive. The method described herein makes it possible to use the ability to analyze F1 seeds with markers to precisely determine their parental origin, using F1 seeds that can be considered as obtained from a simulation of a crossing block on a very small scale.

The Applicant thus presents a new method for assessing the suitability of cereal lines as pollinators (their pollinating ability). The method is based on the analysis of F1 seeds that have been obtained after pollination of male-sterile female tester lines by a pool of male lines. Many male candidates can thus be tested at once for their cross-pollination ability in one single crossing block. Furthermore, pooling the males would lead to competition between their pollinating ability, thus making it possible to intensify any difference between them in their "ability to give seed set" in the context of implementation of the method. The produced F1 kernels are analyzed with the same set of molecular markers that has been designed to individually discriminate the parental lines (male and female lines), enabling the inference of F1 pedigrees produced by a pollen cloud of bulked males. The relative contribution of each male corresponds to their relative "ability to give seed set" (the more F1 seeds originate from a given male parent, the more the male parent has the "ability to give seed set").

Implementation of this method thus does not necessitate much space in the field and it can be performed with a relatively low number of seeds from each male line (present in the pool) making it possible to be applied in early breeding stages. It can also be repeated with different male-sterile females, thereby strengthening the "ability to give seed set" qualification that can be assigned to a given male.

The measured trait "ability to give seed" shows very high heritability and the data generated with this method can be directly used for the selection of genotypes with superior cross-pollination ability.

Depending on the number and relatedness of male components (diversity set with sufficient number of males, bi-parental or multi-parent populations of males) and with an appropriate applied field design, it is possible to use the results obtained by implementation of the method (identification of the best pollinating lines) for analyzing the genetic architecture of this trait "ability to give seed set" by genome-wide association studies or by linkage mapping. The data can be also used to train genomic prediction models. Depending on the used hybrid system, the harvested F1 kernels can be multiplied and hybrid performance and combining ability can be assessed in F2 stage.

The invention thus relates to a method for classifying male plant lines of a pool of male plant lines for their pollination ability, comprising

a) Providing a population of F1 plants obtained by pollinating a male-sterile female plant line by the pool of homozygous male plant lines, wherein the male lines of the pool of male plant lines and the male-sterile female plant line are individually distinguishable by a set of markers,
b) Obtaining material samples from the plants in said population of F1 plants
c) Determining, for each plant of the population of F1 plants, the paternal parent, using the set of markers,
d) Determining, for each male plant in the pool of male plant lines, the proportion of offspring plants in the population of F1 plants,
e) Classifying the male plant lines of the pool of male plant lines for their pollination ability according to the proportion hereby determined.

In some embodiments, the markers of the set of markers are molecular markers (epigenetic markers such as DNA methylation or histone modifications (that are associated with gene expression differences) are included in the molecular markers). These markers are very preferred. However, it may also be possible to use other inheritable markers such as isozymes (presence of variants of proteins detected by electrophoresis), presence or amount of specific metabolites,

In some embodiments, the material samples from the plants of the population of F1 plants are DNA samples.

In preferred embodiments, the plants are autogamous plants, in particular as listed below.

The population of F1 seeds on which the marker analysis is performed may be a subpopulation of the whole population of F1 seeds harvested after the fertilization of the male-sterile female plants. The number of F1 seeds that are analyzed can be determined by the person of skill in the art to allow statistically significant analysis of the proportions of the male parents in the F1 population. It would thus depend notably on the number of different male lines included in the pool of male lines. When using 10 to 30 different males, it may be sufficient to analyze between 150 F1 and a few thousands (such as 4500-5000 F1 seeds), although there is not real upper limit. Testing at least 150 F1 seeds, at least 180 seeds, at least 250 seeds may be sufficient.

The seeds on which the analysis is performed are called F1 seeds or offspring seeds. They are harvested on the female lines, and result from the pollination of such male-sterile female lines by the pool of homozygous male plant lines.

After determining the parental origin for each F1 seeds, it is possible to determine the proportion of F1 seeds having a given male parent, in the population of F1 seeds. Thus, one can obtain, for each male plant in the pool of male plant lines, the proportion of offspring (seeds having this male as a parent) in the population of F1 seed. One can thus classify the male autogamous plant lines of the pool of male wheat autogamous plant lines for their pollination ability according to the proportion hereby determined, the higher the proportion, the higher the pollination ability.

The process is preferably performed with autogamous plant lines, although it can also be performed on semi-autogamous plant lines or allogamous plant lines. The description below focuses on the autogamous plant lines, but every aspect can be implemented with semi-autogamous plant lines or allogamous plant lines.

The process herein disclosed thus makes it possible to select the best pollinating lines from the pool of male autogamous plant lines. These best lines can then be used for further characterization (notably genetic characterization), or for implementing breeding schemes.

The pool of male autogamous plant lines thus comprises

- multiple individuals of
- multiple different male autogamous plant lines. Generally, the pool comprises at least 5, 10, 15, 20, 25, 50, 75, 100, 150, 200, 250 different male autogamous plant lines. However, it is conceivable that the pool comprises higher number of different male autogamous plant lines.

The number of plants of each different male present in the bulk of male plants that is used for pollinating the male-sterile female plants is generally at least 10, at least 20, at least 25, at least 50.

An "autogamous" plant is a plant that is able to perform self-pollination (autogamy), *i.e.* pollination in which pollen from the anthers of a flower is transmitted to the stigma of the same flower, and that would predominantly self-pollinate. The majority (generally more than 90%) of ovules are fertilized through self-pollination, in controlled settings (in the presence of other pollinating plants that would compete with self-pollination).

As autogamous plants, one can cite autogamous cereals, in particular oats, wheat, barley, rice, spelt, triticale, millet, rye, or rape. One can specifically mention oat (*Avena sativa* comprising byzantine; *Avena nuda; Avena strigosa*), barley (*Hordeum vulgare*), rice (*Oryza sativa*), wheat (*Triticum aestivum*), durum wheat (*Triticum durum*), spelt (*Triticum spelta*) and triticale (*Triticosecale*).

One can also cite other plants such as pea (*Pisum sativum*), soybean (*Glycine max*), common bean (*Phaseolus vulgaris*), chickpea (*Cicer arietinum*), lentil (*Lens culinaris*), or tomato (*Solanum lycopersicum*) or eggplant (*Solanum melongena*).

In contrast, allogamous plants rely on cross-pollination (allogamy), where fertilization occurs between the gametes of two different plants of the same species. This can also be determined in controlled settings, where plants would produce significantly fewer seeds or fruits in the absence of external pollinators than in the presence of external pollinators. As examples of allogamous plants, which predominantly rely on cross-pollination for reproduction, and may exhibit traits such as dioecy, self-incompatibility, or reliance on external pollinators, one can cite maize (*Zea mays*), rye (*Secale cereale*) or carrot (*Daucus carota*).

One can note that some plants are partially autogamous plants, and rely on a combination of self-pollination (autogamy) and cross-pollination (allogamy). As partially autogamous plants, one can cite sunflower (*Helianthus annuus,* essentially allogamous, but autogamous under specific conditions), cotton (*Gossypium spp.* essentially autogamous, with cross-pollination performed by insects), tomato (*Solanum lycopersicum*) (allogamous under specific conditions), and *Brassica* species (essentially autogamous, with cross-pollination performed by insects).

One can note that each plant species would have both autogamous and allogamous ability, but would have predominantly one property. Allogamous ability makes it possible to ensure genetic diversity, where autogamous ability makes it possible to have offspring even in the absence of exogenous pollinator plants. The classification of plants as "autogamous" or "allogamous" is known by the person skilled in the art.

The method is preferably performed on autogamous or partially autogamous plants. Indeed, these plants generally have little ability to pollinate other plants (as the pollen would be used to fertilize the ovules of the same plant). These plants generally have anthers and stigma located in close proximity, often within the same flower, facilitating self-pollination. In the context of making hybrids from these plants, it is thus needed to identify these autogamous plants that present the best ability to produce pollen with fertilizing ability. The method is thus perfectly adapted for detecting the ability of a given male plant to properly fertilize other plants.

As indicated above, the method can be performed on autogamous or partially autogamous plants. It is however not discarded to perform this method on allogamous plants, in order to identify and select best pollinator for hybrid breeding programs. In particular, the method could be performed with maize plants.

A "plant line" refers to a genetically distinct and homozygous group of plants of a given species that share specific, stable, and heritable characteristics. The homozygosity of the line ensures uniformity and consistency in the expression of its traits across successive generations. Typically developed through breeding, selection, or genetic engineering, a plant line is defined by its genetic stability and reproducibility, the plants exhibiting at least one specific trait, such as agronomic performance, disease resistance, or quality attributes.

A "male plant" is a plant that can pollinate female plants of the same species, and that thus produces pollen.

A "female plant" is a plant that can be pollinated and fertilized by the pollen of a male plant. After fertilization by male pollen, the plant will produce seeds or fruits (depending on the plant species).

A "male-sterile female plant" is a plant that is a female plant and that has lost its ability to produce pollen. Consequently, a male-sterile female plant would be fertilized by the pollen of another male plant. Various methods exist to induce male sterility. One can cite:

- Cytoplasmic Male Sterility (CMS), which is caused by specific interactions between nuclear and mutated mitochondrial genomes, resulting in inability to produce functional pollen. Such sterility is stably inherited due to its origin in the maternal cytoplasm.
- Genetic Male Sterility (GMS), which is controlled by appropriate nuclear genes, and is thus generally recessive. In particular, fertility can be restored if the male-sterility gene is complemented by a fertile allele. Several nuclear genes responsible for male sterility have been identified in wheat. Some notable examples include ms1, which leads to defective pollen development, ms2, where pollen wall formation is disrupted, ms5 or ms6, additional loci linked to male sterility. Other genes (ms45 or the like) are also known.
- Male Sterility induced by chemical hybridizing agents (CHAs), where chemical agents can

    ◦ inhibit microsporogenesis and prevent the formation of viable pollen by disrupting meiosis in microspore cells (Methyl Benzimidazole Carbamate (MBC), Trifluoromethanesulfonamide (TFMS))
    ◦ Disrupt pollen wall formation, rendering pollen non-functional (such as 2-Hydroxy-5-nitrobenzyl Bromide or cycloheximide)
    ◦ alter hormonal pathways (such as gibberellin, auxin, or cytokinin levels) required for normal anther and pollen development (one can use Ethephon, gibberellin synthesis inhibitors (such as paclobutrazol)
    ◦ have cytotoxic effects such as causing cell death in developing anthers (one can cite sodium methyl arsenate or maleic hydrazide)

- Environment-Sensitive Male Sterility (EGMS), where fertility is influenced by specific environmental factors. Genes involved in EGMS in wheat include tms5, which is linked to Thermo-Sensitive Male Sterility where male sterility is induced by temperature.

Although the CMS and GMS methods would generally provide completely sterile plants, there may be some leakage, notably with the CHA or EGMS methods, but also with these CS and GMS methods: in other words, some plants may produce pollen, although generally in a smaller amount than non-male-sterile plants. When the F1 seeds are analyzed, such leakage would be detected and the F1 seeds resulting from the self-pollination of leaking male-sterile plants would be discarded.

The method thus makes use of F1 plants or seeds that were harvested from the male-sterile female plants that had been exposed to the pollen of the pool of the male plants that are to be tested. The method thus comprises the technical steps of characterizing the F1 plants or seeds, by use of molecular markers (through DNA amplification, markers determination and genome analysis and comparison, usually computer-implemented). The method does not comprise any step of crossing plants that would take place before or after the steps specifically recited above.

"Molecular markers" are segments of DNA associated with specific regions of the genome that can be used to identify genetic variation within and between plant lines. Common types of molecular markers include microsatellites (SSRs), single nucleotide polymorphisms (SNPs), and amplified fragment length polymorphisms (AFLPs). SNPs are currently generally used in modern studies for their abundance and high resolution. Epigenetic markers (such as the presence of methylation at specific locations of the genome) are also envisaged.

In the present methods, the male lines are generally not closely related, and the number of polymorphic molecular markers that are needed to be able to differentiate them does not need to be very high. It is thus generally possible to use between 13 and 20 polymorphic molecular markers to individually characterize each male parent line. When the genome of the male-sterile females is also known (this is preferred), when performing the method herein disclosed, the number of markers is appropriate when it makes it possible to discriminate the male and female lines. This would thus allow to determine, from the genome of the F1 seeds or plants, the paternal chromosomes from the maternal chromosomes.

If the genome of the male-sterile females is not known (such embodiment not being preferred), the number of polymorphic markers to be selected may need to be higher to be able to assign the paternal origin of the F1 seeds in the absence of knowledge of the maternal genome.

To characterize a plant line, one can follow the following protocol: DNA is first extracted from the plant tissue (which may be the F1 seed, F1 half-grain, or a tissue from a plant or plantlet grown from the seed). It is used as a template for PCR amplification of the genomic regions containing the selected markers. The amplified DNA fragments are then characterized using any technique appropriate for the marker type (one can cite gel electrophoresis for SSRs or sequencing, including sequencing, notably next-generation sequencing (NGS) for SNPs; one can also use a DNA chip, such as a DNA chip that is able to differentiate SNPs at various locations of the genome of the plant). The resulting genetic profiles are then analyzed using a computer to create the marker map of the plant, which is then compared to the ones of the male parent lines, or to compute conservative genetic distance between F1 and male parent, thereby making it possible to assign a male parent to the F1 seed or plant.

Although it is possible to use the pool of male plants to pollinate heterozygous male-sterile female plants, it is preferred when the male-sterile female plants are male-sterile female lines (*i.e.* are homozygous).

In some embodiments, the pool of male plants was used to pollinate a pool of male-sterile female plants. In this case, a higher number of molecular markers (several hundreds or thousands of markers) can be used to infer the conservative genetic distance between F1 and males. The female marker profile is not needed in this case. However, it is preferred when the male plants were used to pollinate a single line of male-sterile female plants. The resulting F1 seeds or plants thus all share the same maternal genome and only differ by the pollinating male genome. This makes the genome analysis easier, and allows to obtain specific information as to the effect of the female and flowering window on the pollination ability of males. Furthermore, the conclusion as to the seed-giving ability of the males would only depend on the male genome (the female plants all have the same genome) so that it makes it easier to conclude on this character. It is possible to perform the method with different male-sterile female lines, for instance male-sterile lines from different genetic backgrounds, or presenting different flowering time (nicking), so that the "ability to give seeds" of the males can be assessed depending on the nature of the female line. Indeed, some males can be very efficient in pollinating early-flowering females, while not appropriate for late-flowering females. Using such different female lines would make it possible to refine the knowledge of the ability to give seeds for the males of the pools. In this embodiment, it is possible to classify the male lines' pollinating ability according to each flowering date.

In some embodiments, the pool of male lines is used to pollinate plants of a single male-sterile female line.

In some embodiments, the pool of male lines is used to pollinate plants of a pool of multiple male-sterile female lines (generally from 2, 3, 5 or 10 different male-sterile female lines, rarely more).

In this case, a SNP chip (containing few hundreds or thousands of molecular markers) can be used and analysis would measure conservative distance to the males. This allows to assign F1 profiles to males.

One can thus summarize a preferred process as follows:

- Provide F1 seeds (or plants) being offspring of a male-sterile female line fertilized by a pool of male lines that one wishes to characterize, wherein each male line and the male-sterile female line can be differentiated (individualized) from one another, using a set of markers (notably molecular markers)
- Isolate sample (notably DNA) from the F1 seeds or plants, and use the set of markers on the isolated sample to obtain a profile of each F1 seed or plant
- For each F1 seed or plant, determine the male parent, by comparing the profile of the F1 seed of plant to the profiles of the male plants. In some cases, it is not possible to determine a male parent, and the data for the F1 seed or plant will be discarded (or noted as "not usable").

- For each male parent, counting the number of F1 seeds or plants that originate from this male parent.
- Calculating the frequency of the F1 seeds or plants that originate from each male parent among the F1 seeds or plants that have not been considered as "not usable".

The male parent that has the highest frequency is considered to have the best "ability to give seeds" as compared to the other male plants from the pool. Although one could believe that the "ability to give seeds" may depend on the field conditions, the examples show that this property is conserved between experiments.

To improve the quality of the data, it is preferred to perform replication of the pollination of the females by the males in the male pool. Each replication can thus be individually analyzed, and the various replication or environment (year x location) data would be used for obtaining quantitative genetic parameters such as heritability. Repeating the methods with F1 seeds obtained from different locations, and on different years would allow identification of good pollinator lines, that maintain the ability in different environments and remain such overtime.

In one embodiment, the male-sterile female plants present Cytoplasmic Male Sterility (CMS). In another embodiment, the male-sterile female wheat plants present Genetic Male Sterility (GMS). In yet one embodiment male-sterility is induced by use of CHA in the female plants. In another embodiment, the male-sterile female plants present Environment-Sensitive Male Sterility and the pollination is performed under environmental conditions that trigger male-sterility.

As indicated above, the male parents from the pools can be individually distinguished from each other by molecular (or genetic) markers. Furthermore, it is preferred when the set of markers also makes it possible to distinguish the male lines from the male females. This can ease the analysis of the F1 seeds to obtain the parents.

This embodiment is specifically useful when one uses a pool of different male-sterile female plants. In this case, the female plants of the pool (the population) of female plants are individually distinguishable by molecular markers.

In this embodiment, it may be interesting to determine both the paternal patent of the F1 seeds or plants and the maternal parent.

To obtain appropriate statistics, it is advisable that the pool of male plants contains the same number of seeds adjusted for germination rate for each male line. The method summarized above implied such embodiment. In other embodiments, the number of seeds of each line may be different, but should be known and taken into consideration to restate the statistics (the frequency calculating above should be adjusted, taking into account the frequency (relative weight) of each male line in the male pool).

The design of the pollinating experiment will be performed by the person of skill in the art, taking into account the following elements:

- The males' lines in the male pool should be classified in the same flowering date group (i.e. that the flowering time for the male and female plants should be within 0-5 days). In wheat, flowering time typically refers to the stage when 50% of the plants in a population have reached anthesis. In wheat, one can define an early-flowering group (plants flower earlier in the growing season), an intermediate-flowering group (plants flower midway between early and late groups) and a late late-flowering group (plants flower later in the season).
- The female line (or lines) should be in the same flowering date group than the males (in order to favor that there are stigmas able to receive the pollen of the males
- The size of the female line (or lines) should be a little shorter than the size of the male lines (the pollen being transported by the wind, it would fall by gravity during before reaching the stigmas)
- The orientation of the blocks or rows will preferably take the orientation of the dominant wind into consideration so that the male pollen will be transported to the female lines
- The distance between the males and the female lines should be adapted to the pollen weight, so that the pollen is actually able to reach the female plants.

One of skill in the art is aware of the flowering group of the male or female lines, or of the size of the plants. Knowledge of this information could be a prerequisite before performing the method herein disclosed. Broad knowledge of the plants that are used to obtain the F1 population would indeed be important to make sure that the results are reliable and informative.

Synchronization of flowering would play a role in the interpretation of the results. More robust results may be obtained using several female testers with different flowering times (although they need to be able to be pollinated by the males so that their flowering time should be adapted to the one of the males). Indeed, a male plant may be considered as not having "ability to provide a seed set" if used with female plants with delayed flowering time.

To perform the pollination of the male-sterile females with the males of the pools, so that the F1 population needed for implementing the analysis method is obtained, one can thus envisage sowing one or more blocks of female plants, surrounded on the right and the left by male plants. The number of seeds that are sowed in the bulk of male plants and in the bock of female plants depends on the design of the field experiment and on the number of seeds of male-sterile female plants.

The width of the female plants' blocks should preferably be between 20 cm and around 1 m, so as to make sure that the females in the middle of the blocks can be fertilized. However, width can be larger. Notably, width would be 1.55m if a standard yield plot drilling machine is used. One can note that, if the pollen cannot reach the middle of the female block, the female plants won't be pollinated and no seed will be harvested. The width of the female block is thus not limited and can thus be several meters. Such large widths does not harm the experiment and could enable an easier field design using appropriate existing machines.

The male plants blocks would be around the same size and should be no more than 1-1.5m from the female block. The distance of male plants to female plants could be much shorter (such as 20cm) if CMS or GMS is used an no gametocide application is required. It is also to be noted that when the width of the male plants is too large, this may impair the ability of the most distant plants to pollinate the female plants. However, since the male seeds are all mixed before sewing, a large width does not modify the odds of the plants to pollinate the female plants.

The length of the block can be several meters long.

The density of the female and male plants can be adapted by one of skill in the art. A density between 150 and 400 plants / m$^2$ would be adapted for wheat. Density of the female tester may be higher than the density of male pool. This would result in less tillering of female plants and thus in a slightly earlier flowering which may be favorable.

It is preferred when the design of the pollination scheme of the population of male sterile female wheat lines by the male wheat lines is such as to allow potential pollination of each female line by each male line. Thus the width of the female rows is not too wide, and the male plants are close enough to allow pollen to be transported to the plants. Thus, the female plants in the population of the female wheat plants are exposed to pollination

In some embodiments, the male and female plants are sown together. This is not preferred as this would need more analysis of F1 kernels to identify the ones that are offspring of males and male-sterile females.

In some embodiments, each female line was planted in an individual (a specific) plot and wherein the plants of the pool of male lines were planted in rows adjacent (preferably on each side) to the female plots.

In summary, one can cite a method for classifying male plant lines of a pool of male plant lines for their pollination ability, comprising

a) Providing a population of F1 plants obtained by pollinating a male-sterile female plant line by the pool of homozygous male plant lines, wherein the male lines of the pool of male plant lines and the male-sterile female plant line are individually distinguishable by a set of markers,
b) Obtaining material samples from the plants in said population of F1 plants
c) Determining, for each plant of the population of F1 plants, the paternal parent, using the set of markers,
d) Determining, for each male plant in the pool of male plant lines, the proportion of offspring plants in the population of F1 plants,
e) Classifying the male plant lines of the pool of male plant lines for their pollination ability according to the proportion hereby determined.

In one embodiment, the markers are genetic markers.

In one embodiment, the parental parent of the F1 plants is determined by sequencing or by analysis of the DNA genome of the F1 plants on a DNA chip for determining SNPs.

In one embodiment, the plant is an autogamous plant.

In one embodiment, the autogamous plant is an autogamous cereal selected from oat, barley, rice, wheat, durum wheat, spelt and triticale.

In one embodiment, wherein the male-sterile female plants are male-sterile female lines, in particular presenting Cytoplasmic Male Sterility (CMS), Genetic Male Sterility (GMS), or the male sterility of the female plants being induced by chemical hybridizing agents.

In one embodiment, the set of markers allows to individually distinguish the female plants and to distinguish them from the male plants.

In one embodiment, the pool of male plants contains at least 10 different male lines. In one embodiment, the male-sterile female wheat plants contain plants from between 1 and 5 different lines.

In one embodiment, the male sterile female plants and the male plants have synchronous flowering times.

in one embodiment, the pollination scheme of the population of male sterile female wheat lines by the male wheat lines was designed to allow potential pollination of each female line by each male line.

## DESCRIPTION OF THE FIGURES

Figure 1: Illustration of the design of a field experiment. Two rows of female plants are surrounded by two rows of a pool of male plants.

Figure 2: Contribution and 95% confidence intervals of each male in each set of analyzed F1 kernels. A-C Mid-early CMS female 1 line. Left and right panels represent two repetitions. D-E Mid-late CMS female 2 line. Left and right panels represent two repetitions.

Figure 3: Illustration of a simulation experiment. Expected least significant ratio based on the varying number of marker analyzed F1 kernels (384, 768, 1152 or 1536 progenies) and number of males used in a mix (2 to 200).

Figure 4: Ability to give seed set in a trial. Ability to give seed set score for each female (light or darker grey) and a mean across females (darker grey).

Figure 5: Scatter plot showing the correlation between ability to give seed set and anther extrusion.

Figure 6: Series of 1.5m blocs sown in 6 headrows from the early group. The pollinator mix is sown in headrows 1, 2, 5 and 6. The females are sown in headrows 3 and 4.

Figure 7: percentage of F1 obtained after analysis of F1 harvested after pollination with different males. A result for Group 1. B. Result for Group 3. C Result for Group 7.

## EXAMPLES

All experiments and field trials were performed in private premises.

## Example 1. Measure of the ability to pollinize of multiple males.

Thirty (30) winter wheat lines from the Central European germplasm were selected to establish three male mixes containing 10, 20 or 30 males

- Male mix 1 contains 10 genotypes,
- male mix 2 contains all male mix 1 genotypes plus 10 further genotypes,
- male mix 3 contains all genotypes from male mix 1 and 2 plus 10 further genotypes.

Each male mix contained the cultivar PIKO which is known for its good cross-pollination ability.

A seed counting device was used to ensure equal contribution of each male in the respective male mix.

Two CMS female lines were used as female testers to ensure a broad nicking window.

- CMS female 1 has a mid-early flowering time,
- CMS female 2 has a mid-late flowering time.

In total, 12 crossing blocks were drilled: Each male mix was crossed with each CMS A-line in two replications (3 mixes x 2 females x 2 replications = 12 crossing blocks).

Replications within one block were separated by rye to have an isolation and to facilitate easier visual identification in the field.

A standard single row seeder was used for drilling the crossing blocks, each of which contained six rows. 10g of seeds were used for each row. The two middle rows contained the CMS A-line. The two rows left and right of the two middle rows contained the male mixes.

Each row had a length of 75cm, and the distance between each row was 19cm. Distance between each plot in a block was 50cm.

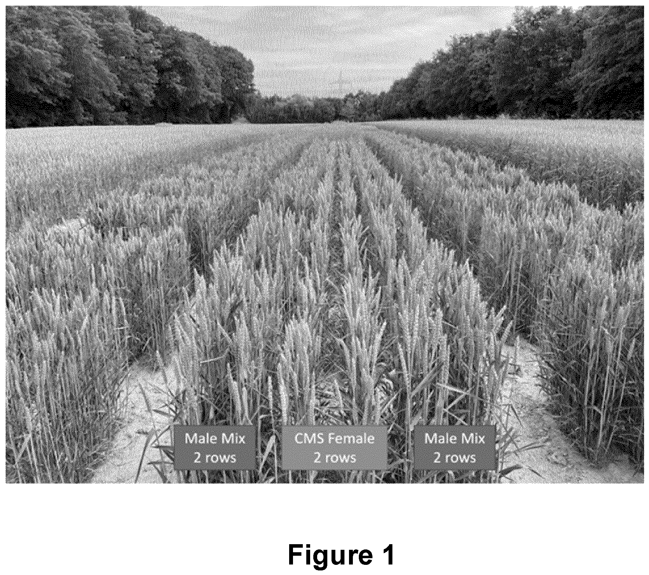

Nine plots represented one replication and two replications were isolated using two plots of rye. Therefore, one block comprised 20 plots (Figure 1).

The 12 blocks crossing blocks were surrounded by two known wheat cultivars which were not part of the experiment.

Before anthesis, five female ears within each replication were isolated by bags to check sterility. Male rows were removed after anthesis to avoid contamination with female rows during harvest.

Female rows of each replication were harvested separately using a sickle.

A random sample of 372 F1 kernels from each replication was then sent for genotyping using 16 highly polymorphic SNP markers covering the following wheat genomes: 1A, 1B, 1D, 2A, 2B, 2D, 3A, 3B, 3D, 4A, 5A, 5B, 6A, 6B, 7A, and 7B. All 30 males and the two CMS females were genotyped with the same set of markers. All potential F1 marker profiles were calculated based on male and female marker information. The marker data of the genotyped F1 kernels was then used to assign the respective F1 pedigrees. F1 profiles with no clear match due to the two additional surrounding wheat cultivars were discarded from the analysis.

The contribution of each male within each replication was calculated.

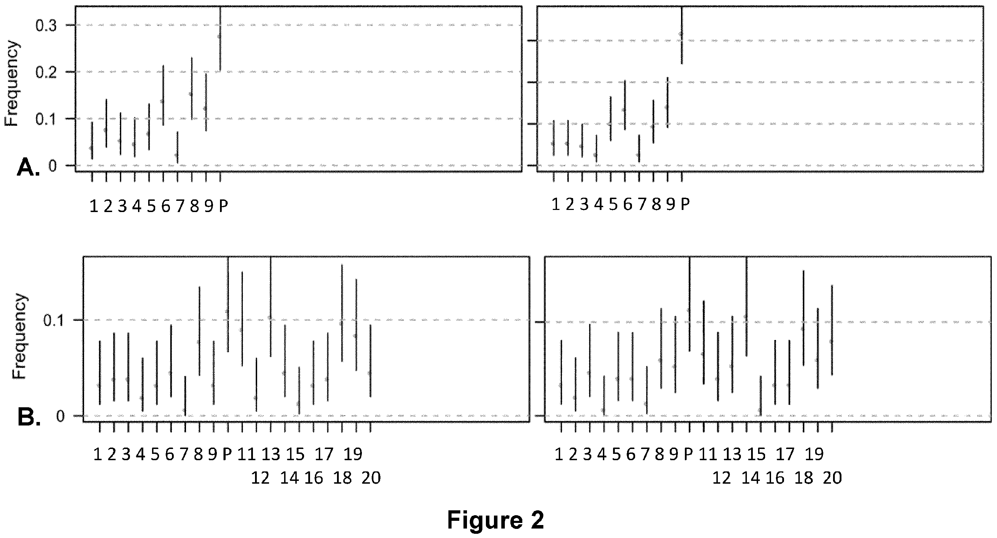

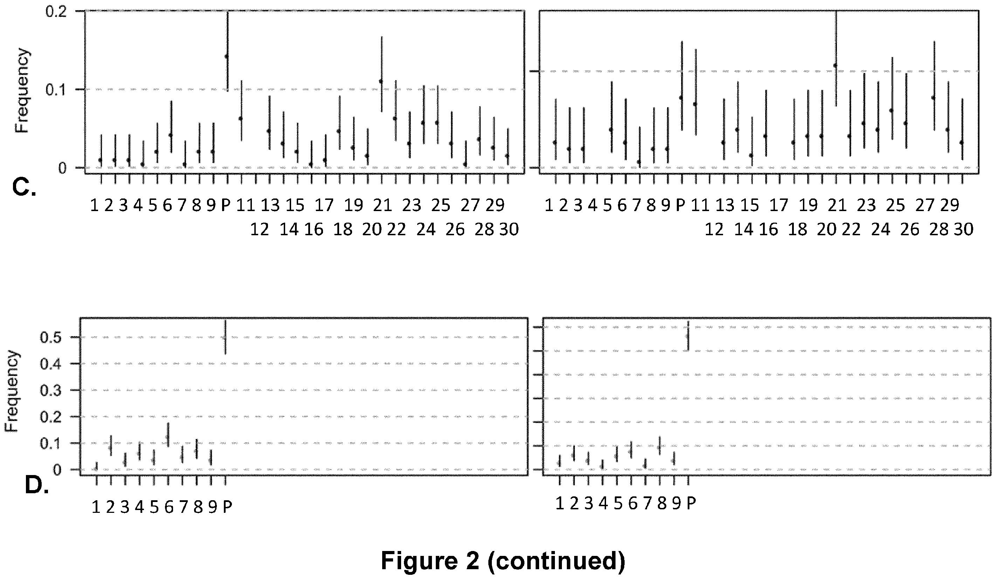

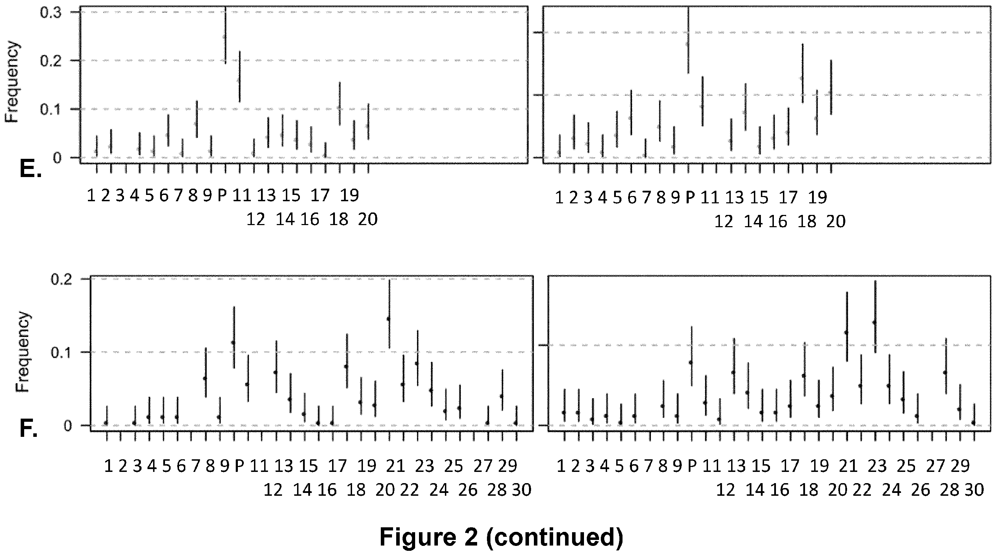

Figure 2 shows the contribution of each male in each crossing block. The visual assessment of two replications for a respective CMS female and male mix show a consistent pattern and clear variation between the different male candidates.

This is supported by the analysis of repeatability (heritability of a single location) of the trait "ability to give seed set" for a respective female and male mix combination which were in a range of 0.82 to 0.91 (Table 1). Repeatability was calculated as $h^2 = 1 - \frac{\overline{\vartheta}_{BLUP}}{2\sigma_G^2}$ where $\overline{\vartheta}_{BLUP}$ is the mean variance of a difference of two BLUPs and $\sigma_G^2$ is the genotypic variance (Cullis et al. 2006).

Table 1: Repeatability of the trait "ability to give seed set" in first trial.

| Female | Male | Repeatability |
| --- | --- | --- |
| Female 1 CMS | Male Mix 1 | 0.89 |
| Female 1 CMS | Male Mix 2 | 0.82 |
| Female 1 CMS | Male Mix 3 | 0.82 |
| Female 2 CMS | Male Mix 1 | 0.87 |
| Female 2 CMS | Male Mix 2 | 0.91 |
| Female 2 CMS | Male Mix 3 | 0.89 |

In total, 4352 F1 kernels were genotyped and 54% of these could be assigned to one of the potential male lines. The remaining 46% belong to one of the two surrounding wheat cultivars which were not part of the experiment.

There is a clear effect between the mid-early and mid-late CMS female lines. The cultivar PIKO confirmed its very high cross-pollination potential with both female testers, but some male perform differently depending on the flowering date.

Another trial was performed the following year.

161 male lines were tested in front of two CMS female tester.

The trial was allocated in strips using yield plot sizes of male mixes and female testers

Each cell is representing a standard yield plot (7.2m$^2$) instead of using a Hege rack drilling machine. Female plots were harvested with a combine and a 200g sample of each plot was taken.

Afterwards, a random sample of 4650 seeds of each female was genotyped using again the same 16 polymorphic markers. Due to the higher number of male lines and the limited number of markers, not all F1 seed could be assigned without error. 2889 and 3183 F1 kernels could be assigned correctly, respectively. The estimated repeatability was on a similar level compared to the previous trial (Table 2).

Table 2 Repeatability of the trait "ability to give seed set" in another trial

| Female | Male | Repeatability |
| --- | --- | --- |
| Female 1 CMS | Male Mix | 0.81 |
| Female 2 CMS | Male Mix | 0.79 |

## Example 2. Simulation experiment

A simulation and analytical estimation of the experiment was conducted to assess the power of the method described in example 1.

Considering a male with an average True Pollination Rate of 1/n (n the number of male in the experiment), its Observed Pollination rate will follow a binomial distribution X0 ~B(m,1/n) where m is the number of F1s (and n the number of males). Another male significantly better than the average male should have a True Pollination Rate of k/n (k>0) and follow a binomial distribution X1 -B(m,k/n). It is possible to find, depending on m and n, the least significant k values so that the OPR of the second male is higher that the OPR of an average male 95% of the time.

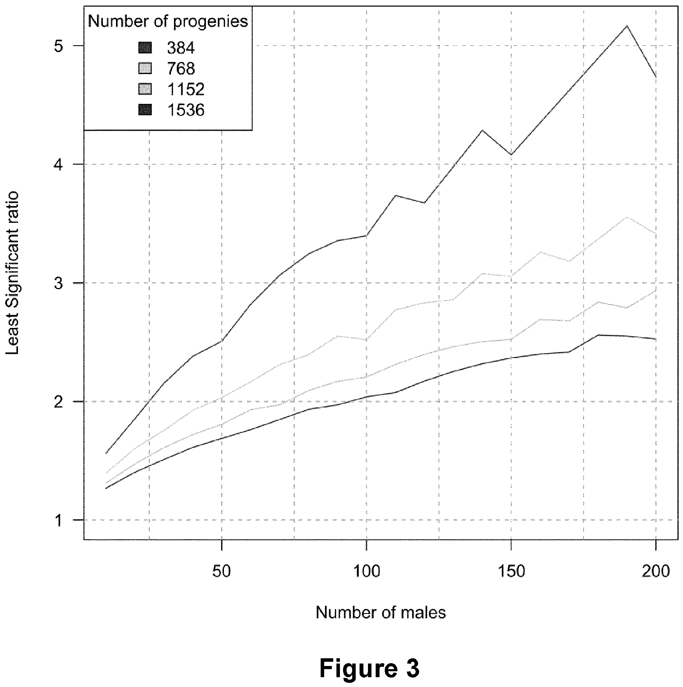

This least significant ratio has been calculated in dependence of different numbers of F1 progenies (384, 768, 1152 or 1536) and males used as a mix (2 - 200). For instance, with 50 males and 384 F1s, each male may appear 1/50th = 2% in the progenies, only males at least 2.5 times more productive will have higher OPR 95% compared to an average male (Figure 3). This example provides information can be used to determine the number of F1 that need to be tested.

## Example 3. Field trial

Another trial using 200 males in a mix and two females was implemented.

A standard wheat yield plot drilling machine (7.2m² per plot) was used and the trial design was arranged as shown in Table 3.

Table 3. Trial design using yield plot size

| Male mix | Late female | Male mix | Early female | Male mix |
|----------|-------------|----------|--------------|----------|
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |
| Male mix | Late female | Male mix | Early female | Male mix |

This design allowed a harvest with a combine with sampling. From each female strip, a total of 4650 seeds were randomly sampled and genotyped.

For sake of higher precision, all seeds were genotyped with a SNP chip containing 2681 markers (midplex).

Based on a conservative genetic distance (D=1-N/M, where M=total number of markers, N=number of markers with at least one allele in common between the two individuals) F1 profiles and the ability to give seed set of each male was deduced.

A conservative threshold for genetic distance measure was set to 0.002. F1 kernels with a higher distance were not considered for analysis. This led to 5376 declared F1 profiles out of 9300 samples. For sake of simplicity, the frequency of each male was transformed linearly to a 1 to 9 APS score (1=low, 9=high).

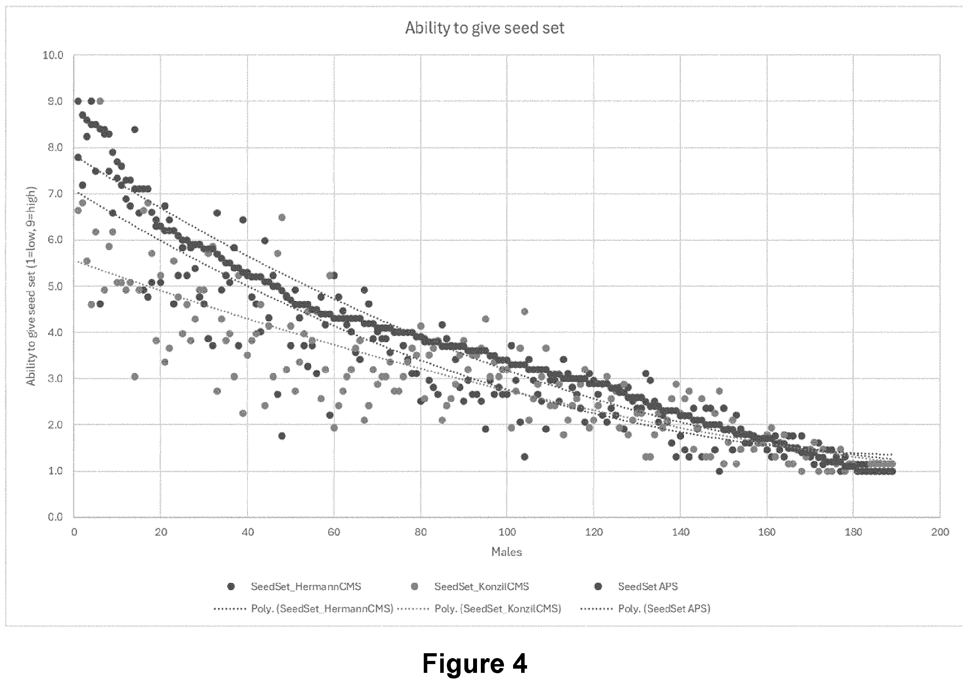

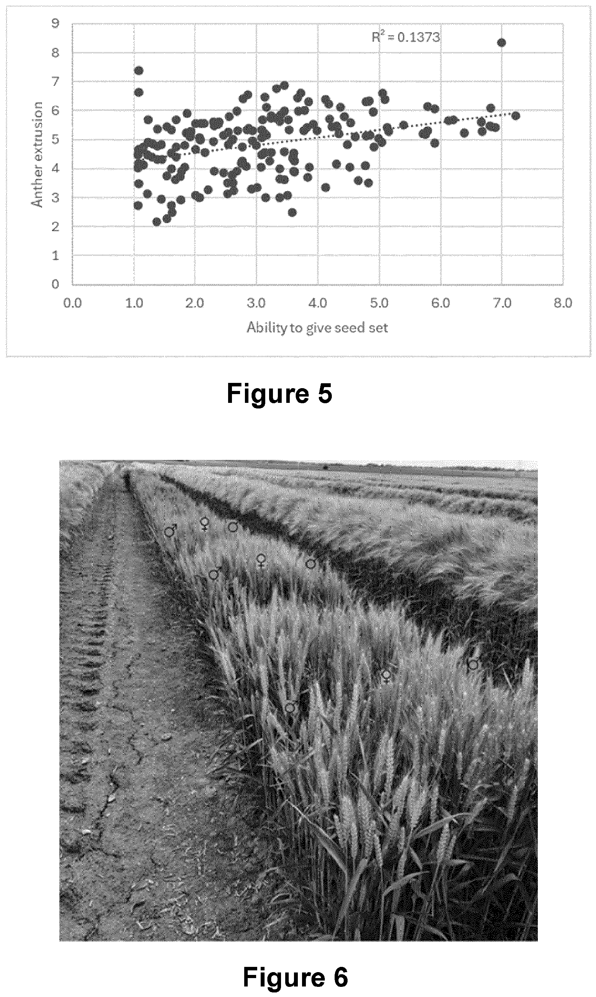

Figure 4 is showing the results for each female tester individually and a mean across females. In other trials, male lines were also evaluated for their anther extrusion and genomic estimated breeding values for assessed. A scatter plot showing the correlation between anther extrusion and ability to give seed set can be drawn (figure 5). This data is revealing just a moderate correlation of r=0.37 and is supporting the hypothesis that anther extrusion is required but is not sufficient to select for the target trait seed set.

## Example 4. Other trials

54 hybrid production plots each made of 6 headrows and being 1.5m long were sown in Autumn 2021 with a nursery seed sowing machine.

27 different combinations each made of single CMS females (headrows 3 and 4) and a mix of 10, 20 respectively 30 pollinators (headrows 1, 2, 5 and 6) were sown in 2 repetitions.

The hybrid production was organized in 3 sub-blocks following 3 earliness groups: early, mid-early and mid-late. The 3 sub-blocs were separated with barley plots which pollen production occurs before the start of the wheat flowering and did not consequently compete with the production of pollen from the wheat pollinators.

The 9 females selected were CMS lines carrying the cytoplasm of *Triticum timopheevii* and thus fully sterile:

- Early: E1 (Early 1), E2 (Early 2) and E3 (Early 3)
- Mid-Early: ME1, ME2 and ME3
- Mid-late: ML1, ML2 and ML3

One of the 90 males selected wad a restorer line.

For each earliness category, 3 male sub-groups were constituted:

- first sub-group with 10 males chosen randomly.
- first sub-group with 20 males chosen randomly.
- first sub-group with 30 males chosen randomly.

In every of the male groups, a pollinator line with a known high anther extrusion (rated 9 in a scale from 0=no extrusion to 9=full extrusion) was added for each earliness category (P-E, P-ME and P-ML).

*Molecular analysis and results*

The females of 48 plots were harvested by hand (with a sickel) at crop maturity stage (from 170g to 1.3kg per plot) and one seed sample per every plot was prepared for molecular analysis. A total of 8 samples was selected for molecular analysis.

- MIX 1 = E1 × 10 males
- MIX 2 = E1 × 20 males
- MIX 3 = E1 × 30 males
- MIX 4 = ME2 × 10 males
- MIX 5 = ME2 × 20 males
- MIX 6 = ME2 × 30 males
- MIX 7 = ML1 × 10 males
- MIX 8 = ML1 × 20 males

For each of these 8 samples, 186 seeds were individually analyzed with a set of 43 markers (PU42 and the marker for Rf1). Not all analyzed seeds could be assigned to a given male, as explained above.

From the analysis, the male lines can be sorted (Tables 4-11).

Line with superior pollinating ability can be easily identified. For example, the line 9 in mixes 7 and 8 always show higher pollinating ability. It is not surprising that some male lines show good pollinating ability in some trials and less good ability in others. This is because the sampling of F1 is low and the standard deviation remains high. However, this example shows that the it is possible to classify the pollinating ability (or ability to gibe seed sets) of males by analyzing the F1 progeny.

Tables 4-6 show the results observed for Mixes 1-3 (early subgroups).

Table 4. Results for Mix 1. Count corresponds to the number of observed F1 for each parent. Expected frequency if the pollinating ability of all lines was identical: 10%

| Line | Count | Proportion |
|------|-------|------------|
| 1 | 4 | 2,4% |
| 2 | 5 | 3,0% |
| 3 | 8 | 4,8% |
| 4 | 8 | 4,8% |
| 5 | 12 | 7,2% |
| 6 | 15 | 9,0% |
| 7 | 16 | 9,6% |
| 8 | 18 | 10,8% |
| 9 | 40 | 24,1% |
| P-E | 40 | 24,1% |

Table 5. Results for Mix 2. Count corresponds to the number of observed F1 for each parent. Expected frequency if the pollinating ability of all lines was identical: 5%

| Line | Count | Proportion |
|---|---|---|
| 11 | 1 | 0,6% |
| 2 | 2 | 1,2% |
| 12 | 2 | 1,2% |
| 1 | 3 | 1,8% |
| 5 | 3 | 1,8% |
| 6 | 3 | 1,8% |
| 3 | 3 | 1,8% |
| 4 | 4 | 2,4% |
| 13 | 4 | 2,4% |
| 14 | 4 | 2,4% |
| 15 | 7 | 4,2% |
| 16 | 9 | 5,4% |
| 7 | 10 | 6,0% |
| P-E | 11 | 6,5% |
| 9 | 15 | 8,9% |
| 17 | 15 | 8,9% |
| 18 | 16 | 9,5% |
| 8 | 18 | 10,7% |
| 19 | 19 | 11,3% |
| 20 | 19 | 11,3% |

Table 6. Results for Mix 3. Count corresponds to the number of observed F1 for each parent. Expected frequency if the pollinating ability of all lines was identical: 3.33%

| Line | Count | Proportion |
|---|---|---|
| 2 | 0 | 0,0% |
| 20 | 1 | 0,6% |
| 21 | 1 | 0,6% |
| 7 | 2 | 1,3% |
| 1 | 2 | 1,3% |
| 4 | 2 | 1,3% |
| 19 | 2 | 1,3% |
| 22 | 2 | 1,3% |
| 23 | 2 | 1,3% |
| 24 | 2 | 1,3% |
| 25 | 3 | 1,9% |
| 26 | 3 | 1,9% |
| 6 | 4 | 2,5% |
| 3 | 4 | 2,5% |
| 15 | 4 | 2,5% |

(continued)

| Line | Count | Proportion |
|------|-------|------------|
| 13 | 4 | 2,5% |
| 16 | 4 | 2,5% |
| 12 | 4 | 2,5% |
| 27 | 4 | 2,5% |
| 28 | 5 | 3,2% |
| 18 | 6 | 3,8% |
| 29 | 6 | 3,8% |
| 8 | 7 | 4,5% |
| 30 | 7 | 4,5% |
| 5 | 8 | 5,1% |
| 14 | 10 | 6,4% |
| P-E | 13 | 8,3% |
| 9 | 14 | 8,9% |
| 11 | 14 | 8,9% |
| 17 | 17 | 10,8% |

Tables 7-9 show the results observed for Mixes 4-6 (mid-early subgroups).

Table 7. Results for Mix 4. Count corresponds to the number of observed F1 for each parent. Expected frequency if the pollinating ability of all lines was identical: 10%

| Line | Count | Proportion |
|------|-------|------------|
| P-ME | 0 | 0,0% |
| 1 | 3 | 2,1% |
| 2 | 5 | 3,5% |
| 3 | 6 | 4,2% |
| 4 | 9 | 6,3% |
| 5 | 12 | 8,3% |
| 6 | 14 | 9,7% |
| 7 | 20 | 13,9% |
| 8 | 31 | 21,5% |
| 9 | 44 | 30,6% |

Table 8. Results for Mix 5. Count corresponds to the number of observed F1 for each parent. Expected frequency if the pollinating ability of all lines was identical: 5%

| Line | Count | Proportion |
|------|-------|------------|
| 1 | 0 | 0,0% |
| P-ME | 0 | 0,0% |
| 4 | 1 | 0,7% |
| 11 | 2 | 1,5% |
| 12 | 2 | 1,5% |
| 13 | 2 | 1,5% |

(continued)

| Line | Count | Proportion |
|------|-------|------------|
| 6 | 3 | 2,2% |
| 3 | 3 | 2,2% |
| 14 | 3 | 2,2% |
| 2 | 5 | 3,7% |
| 15 | 6 | 4,4% |
| 16 | 7 | 5,1% |
| 5 | 8 | 5,9% |
| 17 | 8 | 5,9% |
| 18 | 8 | 5,9% |
| 19 | 13 | 9,6% |
| 7 | 14 | 10,3% |
| 20 | 14 | 10,3% |
| 9 | 15 | 11,0% |
| 8 | 22 | 16,2% |

Table 9. Results for Mix 6. Count corresponds to the number of observed F1 for each parent. Expected frequency if the pollinating ability of all lines was identical: 3.33%

| Line | Count | Proportion |
|------|-------|------------|
| 18 | 0 | 0,0% |
| 21 | 0 | 0,0% |
| P-ME | 0 | 0,0% |
| 1 | 1 | 0,7% |
| 21 | 1 | 0,7% |
| 14 | 1 | 0,7% |
| 22 | 1 | 0,7% |
| 11 | 2 | 1,4% |
| 16 | 2 | 1,4% |
| 23 | 2 | 1,4% |
| 4 | 3 | 2,2% |
| 3 | 3 | 2,2% |
| 13 | 3 | 2,2% |
| 17 | 3 | 2,2% |
| 24 | 3 | 2,2% |
| 25 | 3 | 2,2% |
| 2 | 4 | 2,9% |
| 12 | 4 | 2,9% |
| 26 | 4 | 2,9% |
| 27 | 5 | 3,6% |
| 19 | 6 | 4,3% |
| 6 | 7 | 5,0% |

(continued)

| Line | Count | Proportion |
| --- | --- | --- |
| 15 | 7 | 5,0% |
| 5 | 9 | 6,5% |
| 28 | 9 | 6,5% |
| 9 | 10 | 7,2% |
| 29 | 10 | 7,2% |
| 7 | 11 | 7,9% |
| 30 | 12 | 8,6% |
| 8 | 13 | 9,4% |

Tables 10-11 show the results observed for Mixes 7-8 (mid-late subgroups).

Table 10. Results for Mix 7. Count corresponds to the number of observed F1 for each parent. Expected frequency if the pollinating ability of all lines was identical: 10%

| Line | Count | Proportion |
| --- | --- | --- |
| 1 | 1 | 0,6% |
| 2 | 2 | 1,1% |
| 3 | 3 | 1,7% |
| 4 | 5 | 2,8% |
| 5 | 5 | 2,8% |
| 6 | 7 | 3,9% |
| 7 | 12 | 6,7% |
| 8 | 14 | 7,9% |
| 9 | 26 | 14,6% |
| P-ML | 103 | 57,9% |

Table 11. Results for Mix 8. Count corresponds to the number of observed F1 for each parent. Expected frequency if the pollinating ability of all lines was identical: 5%

| Line | Count | Proportion |
| --- | --- | --- |
| 4 | 0 | 0,0% |
| 3 | 0 | 0,0% |
| 11 | 0 | 0,0% |
| 12 | 0 | 0,0% |
| 5 | 1 | 0,8% |
| 2 | 2 | 1,5% |
| 13 | 2 | 1,5% |
| 14 | 3 | 2,3% |
| 15 | 3 | 2,3% |
| 1 | 4 | 3,1% |
| 16 | 4 | 3,1% |
| 17 | 5 | 3,8% |
| 18 | 5 | 3,8% |

(continued)

| Line | Count | Proportion |
|---|---|---|
| 7 | 6 | 4,6% |
| 19 | 6 | 4,6% |
| 20 | 10 | 7,6% |
| 6 | 12 | 9,2% |
| 8 | 16 | 12,2% |
| 9 | 22 | 16,8% |
| P-ML | 30 | 22,9% |

## Example 5. Other trials

Field experiment

14 production blocks of each 3 yield plots of 6m$^2$ each were sown in autumn 23. In every block, the central plot was sown with a mix of 3 CMS females and the 2 surrounding plots with a mix of 17 to 21 males.

2 groups of 3 CMS females each were formed, the CMS females being in equal quantity in their respective groups:

- Mid early group: ME-F1 + ME-F2 + ME-F3
- Mid-late group: ML-F1 + ML-F2 + ML-F3

7 groups of 17 to 20 pollinators were constituted. The lines forming these groups are lines that possess an anther extrusion rated from 8 to 9 (scale from 0=no extrusion to 9=full extrusion).

- Mid-early groups: GROUP1 to GROUP5
- Mid-late group: GROUP6 and GROUP7

For each of the 9 blocks, 2 repetitions were sown.

At maturity the 18 female blocks were harvested with a plot harvester. 3.7 to 4.9kg were harvested from every single plot.

### Molecular analysis and results

A sample of 186 seeds from each of the 14 harvested female plots was analyzed with a panel of 16 divergent SNPs (PU16). The molecular data set of each of the 2 repetitions were merged. In every of the 7 combinations, it was possible to identify better pollinators, which gave the highest number of seeds in the harvested samples.

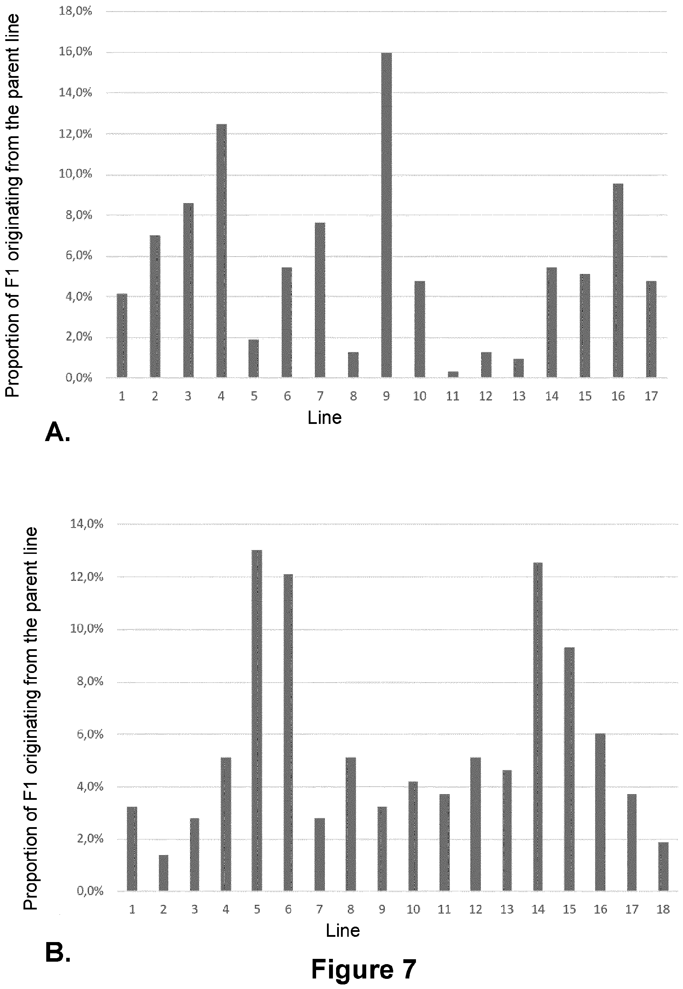

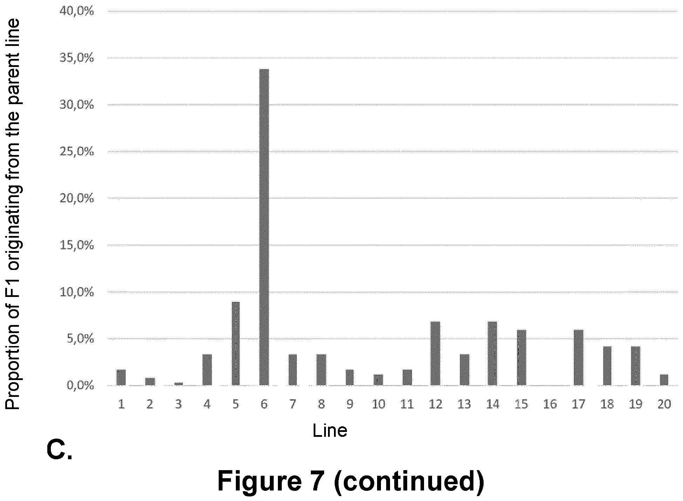

Figure 7 shows representative results of the percentage of F1 obtained after analysis of F1 harvested after pollination with different males for Group 1 (Fig. 7.A), Group 3 (Fig. 7.B) and Group 7 (Fig. 7.C). The highest percentage show a higher ability to give seed set. The same kinds of figures could be obtained for the other groups (not shown).

The method shows high potential to select for genotypes with good cross-pollination ability and shows high flexibility as only needed genotyping of a few hundred or thousand F1 kernels. When using a CMS or NGMS female line, the method allows small-scale crossing blocks combined with manual harvest.

If a larger number of males should be tested, the method can be implemented using yield plots or long strips for males and females, as there is no limitation regarding crossing block size. If half-grains are genotyped, there is further the possibility to multiply the analyzed F1 kernels. The trait "ability to give seed set" showed very high repeatability when detected by the method herein disclosed.

## Claims

1.  Method for classifying male plant lines of a pool of male plant lines for their pollination ability, comprising

    a) Providing a population of F1 plants obtained by pollinating a male-sterile female plant line by the pool of homozygous male plant lines, wherein the male lines of the pool of male plant lines and the male-sterile female plant line are individually distinguishable by a set of markers,

b) Obtaining material samples from the plants in said population of F1 plants

c) Determining, for each plant of the population of F1 plants, the paternal parent, using the set of markers,

d) Determining, for each male plant in the pool of male plant lines, the proportion of offspring plants in the population of F1 plants,

e) Classifying the male plant lines of the pool of male plant lines for their pollination ability according to the proportion hereby determined.

2. The method of claim 1, wherein the markers are genetic markers.

3. The method of claim 1, wherein the parental parent of the F1 plants is determined by sequencing or by analysis of the DNA genome of the F1 plants on a DNA chip for determining SNPs.

4. The method of claim 1 or 2, wherein the plant is an autogamous plant.

5. The method of any one of claims 1 to 4, wherein the male-sterile female plants are male-sterile female lines.

6. The method of any one of claims 1 to 5, wherein the male sterile female plants present Cytoplasmic Male Sterility (CMS).

7. The method of any one of claims 1 to 5, wherein the male sterile female plants present Genetic Male Sterility (GMS).

8. The method of any one of claims 1 to 5, wherein the male sterility of the female plants was induced by chemical hybridizing agents.

9. The method of any one of claims 1 to 8, wherein the set of markers allows to individually distinguish the female plants and to distinguish them from the male plants.

10. The method of any one of claims 1 to 9, wherein the pool of male plants contains at least 10 different male lines.

11. The method of any one of claims 1 to 10, wherein the male-sterile female wheat plants contain plants from between 1 and 5 different lines.

12. The method of any one of claims 1 to 11, wherein the male sterile female plants and the male plants have synchronous flowering times.

13. The method of any one of claims 4 to 12, wherein the autogamous plant is an autogamous cereal selected from oat, barley, rice, wheat, durum wheat, spelt and triticale.

14. The method of any one of claims 1 to 13, wherein the pollination scheme of the population of male sterile female wheat lines by the male wheat lines was designed to allow potential pollination of each female line by each male line.

# Figure 1

Figure 2

Figure 2 (continued)

Figure 2 (continued)

**Figure 3**

Figure 4

Figure 5

Figure 6

Figure 7

C.

# Figure 7 (continued)

Europäisches
Patentamt

European
Patent Office

Office européen
des brevets

# EUROPEAN SEARCH REPORT

**Application Number**

EP 24 30 7324

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KHERDE MADHUKAR K. ET AL: "Cross Pollination Studies with Male Sterile Wheats of Three Cytoplasms, Seed Size on F1 Plants, and Seed and Anther Size of 45 Pollinators", CROP SCIENCE, vol. 7, no. 4, 1 July 1967 (1967-07-01), pages 389-394, XP093287575, US ISSN: 0011-183X, DOI: 10.2135/cropsci1967.0011183X000700040034x Retrieved from the Internet: URL:https://onlinelibrary.wiley.com/doi/pdf/10.2135/cropsci1967.0011183X000700040034x> * page 390, column 1, last paragraph - column 2, paragraph 5 * * page 393, column 2, paragraph 2; tables * ----- | 1-14 | INV. A01H1/02 C12Q1/68 C12Q1/6895 |
| X | D'SOUZA L.: "Studies on the suitability of wheat as pollen donor for cross pollination, compared with rye, Triticale and Secalotricum", ZEITSCHRIFT FUER PFLANZENZUECHTUNG., vol. 63, 1 December 1970 (1970-12-01), pages 246-269, XP093287756, DE ISSN: 0044-3298 * page 607, paragraph 2 - page 611, paragraph 1; tables 5,6 * ----- -/-- | 1-14 | TECHNICAL FIELDS SEARCHED (IPC) C12Q A01H |

The present search report has been drawn up for all claims

| Place of search | Date of completion of the search | Examiner |
|---|---|---|
| Munich | 18 June 2025 | Burkhardt, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches
Patentamt

European
Patent Office

Office européen
des brevets

## EUROPEAN SEARCH REPORT

**Application Number**

EP 24 30 7324

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LANGER SIMON M. ET AL: "Phenotypic evaluation of floral and flowering traits with relevance for hybrid breeding in wheat (Triticum aestivum L.)", PLANT BREEDING, vol. 133, no. 4, 29 May 2014 (2014-05-29), pages 433-441, XP093287640, DE ISSN: 0179-9541, DOI: 10.1111/pbr.12192 Retrieved from the Internet: URL:https://onlinelibrary.wiley.com/doi/pdf/10.1111/pbr.12192> * page 433, column 2, paragraph 4 - page 435, column 2, paragraph 3; figure 4 * ----- | 1-14 | |
| X | SCHNEIDER JOHANNES ET AL: "Assessing the Suitability of Elite Lines for Hybrid Seed Production and as Testers in Wide Crosses With Wheat Genetic Resources", FRONTIERS IN PLANT SCIENCE, vol. 12, no. 689825, 14 June 2021 (2021-06-14), pages 1-11, XP093287703, CH ISSN: 1664-462X, DOI: 10.3389/fpls.2021.689825 * page 2, column 2, last paragraph - page 4, column 2, paragraph 2 * ----- -/-- | 1-14 | **TECHNICAL FIELDS SEARCHED (IPC)** |

The present search report has been drawn up for all claims

| Place of search | Date of completion of the search | Examiner |
|---|---|---|
| Munich | 18 June 2025 | Burkhardt, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

| Europäisches Patentamt<br>European Patent Office<br>Office européen des brevets | **EUROPEAN SEARCH REPORT** | **Application Number**<br>EP 24 30 7324 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MIEDANER THOMAS ET AL: "Effective Pollen-Fertility Restoration Is the Basis of Hybrid Rye Production and Ergot Mitigation",<br>PLANTS,<br>vol. 11, no. 9, 20 April 2022 (2022-04-20), pages 1-17, XP093287624,<br>ISSN: 2223-7747, DOI: 10.3390/plants11091115<br>* page 4, last paragraph - page 5, last paragraph; figure 2 *<br>----- | 1-14 | |
| A | DE VRIES A. PH.: "Some aspects of cross-pollination in wheat (Triticum aestivum L.). 4. Seed set on male sterile plants as influenced by distance from the pollen source, pollinator: Male sterile ratio and width of the male sterile strip",<br>EUPHYTICA,<br>vol. 23, no. 3,<br>1 November 1974 (1974-11-01), pages 601-622, XP093287737,<br>Dordrecht<br>ISSN: 0014-2336, DOI: 10.1007/BF00022483<br>Retrieved from the Internet:<br>URL:https://link.springer.com/article/10.1007/BF00022483/fulltext.html><br>* the whole document *<br>-----<br>-/-- | 1-14 | **TECHNICAL FIELDS SEARCHED (IPC)** |

The present search report has been drawn up for all claims

| Place of search | Date of completion of the search | Examiner |
|---|---|---|
| Munich | 18 June 2025 | Burkhardt, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................

& : member of the same patent family, corresponding document

EPO FORM 1503 03.82 (P04C01)

Europäisches
Patentamt
European
Patent Office
Office européen
des brevets

# EUROPEAN SEARCH REPORT

**Application Number**

EP 24 30 7324

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | R. WHITFORD ET AL: "Hybrid breeding in wheat: technologies to improve hybrid wheat seed production", JOURNAL OF EXPERIMENTAL BOTANY, vol. 64, no. 18, 31 October 2013 (2013-10-31), pages 5411-5428, XP055230612, GB ISSN: 0022-0957, DOI: 10.1093/jxb/ert333 * page 5419, column 1, last paragraph – page 5420, column 1, paragraph 2; figure 4B * | 1-14 | |

-----

|  |  | TECHNICAL FIELDS SEARCHED (IPC) |
|---|---|---|

The present search report has been drawn up for all claims

| Place of search | Date of completion of the search | Examiner |
|---|---|---|
| Munich | 18 June 2025 | Burkhardt, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)